# EUROPEAN PATENT APPLICATION

(11) **EP 4 056 055 A1**
(43) Date of publication of application: **14.09.2022**
(21) Application number: 21161408.6
(22) Date of filing: 09.03.2021
(51) Int. Cl.: A24F 40/05, A24F 40/48

(54) **DEVICE FOR VOLATILISATION OF AN AQUEOUS SOLUTION AND METHOD THEREOF**

(71) Applicant: Avogen Tech SA, 1870 Monthey (CH)
(72) Inventor: Gendre, Yannick, 1926 Fully (CH); Avolio, David, 1868 Collombey (CH)
(74) Representative: P&TS SA (AG, Ltd.)

(57) **Abstract**

The present invention relates to a device (1) adapted to volatilize a viscous liquid composition (S) into microdroplets (5) comprising a pre-heating chamber (12) upstream a volatilizing element. It further relates to a method of providing an aerosol from a liquid composition, aqueous liquid compositions used for the production of aerosols, and to a kit comprising the same.

## Description

### Technical field

The present invention concerns a vaporizing device such as an electronic cigarette or an inhalator. In particular, the device according to the present invention is adapted for the vaporization of aqueous solutions with an improved efficiency. It allows to produce microdroplets having a mean diameter lower than around 4 µm, preferably in the order of 1 µm. The present disclosure further concerns a process for vaporizing an aqueous solution in an aerosol of such droplets. It also concerns a kit comprising the device and at least one aqueous solution separately packed.

### State of the art

It is known that the mean size of the droplets is an important parameter for the efficiency of an inhaled substance. Microdroplets of around 4 µm are usually considered optimal to ingress through the lung. Whether the substance, or the solution wherein it is solved, relates to a pharmaceutical product or to a tobacco derivative such as nicotine, it is preferable to produce an aerosol having homogenous particle size of a diameter lower than around 4 µm.

The substances used for inhalation are often combined with additives or organic solvents for stability or homogeneity reasons. For the purpose of vaporization, such non-aqueous solvents are usually more favorable for producing low size microdroplets due to their low natural viscosity. However, the additives or organic solvents may provide some negative effects. Beside the potential non desirable interaction with the organism, such non aqueous solutions can provide irritations, bad taste, or any other detrimental sensations once inhaled or during inhalation. Aqueous solutions thus appear preferable for the inhalation. Due to the natural Lower viscosity of the water compared to organic solvent, it is difficult to produce an aerosol having the requested particle mean size of lower than 4 µm.

The document WO9501137 describes a device allowing to heat the substance in solution until it vaporizes. Such a method does not appear suitable for aqueous solutions because the high enthalpy of vaporization of the water requests to delivering locally a high amount of energy to vaporize the solvent. This results in a high consumption of energy and of a long laps time before vaporization occurs. In addition, temperature may degrade the substances of the composition, or induce non desirable chemical reaction in the mixture.

The document US2019014819 describes a device allowing to volatilize aerosol precursors by means of a piezoelectric mesh so as to avoid any side effects resulting from a heating process. Although compositions based on organic solvents can be volatilized, such arrangement does not appear directly applicable for aqueous solutions. In particular, it is non optimal for controlling the mean size of the droplets of an aqueous composition. In addition, the piezoelectric mesh necessitates an electrical boost to vaporize the solution, which represent a limitation in term of autonomy, when the device is fed by a battery.

### Brief summary of the invention

It is thus an object of the present invention to provide a device allowing to nebulize an aqueous solution with a controlled droplet size. In particular, it is an aim of the present invention to provide a device allowing to nebulize aqueous solutions in an aerosol of droplets having a mean diameter lower than 4 µm, or in the order of 1 µm or lower.

The aim is also to provide a device allowing to reduce the viscosity of liquid compositions below 1cP so that the flow rate of the resulting aerosol is improved.

Another aim of the present invention is to provide a device allowing to volatilize an aqueous solution without burning or degrading the substances it comprises. It is thus an object to volatilize the aqueous solution without boiling the solution.

Another object of the present disclosure is to provide a process for vaporizing an aqueous solution into an aerosol of droplets having a controlled means size. It is more particularly an object of vaporizing the aqueous solution while controlling the mean size of the particles.

Another object of the present invention is to provide a device adapted for volatilizing an aqueous solution and allowing to modulated the temperature, the size of the emitted droplets or the duration of the volatilization.

It is a further object to provide an aqueous solution comprising at least one substance having biological effects, the aqueous solution having a viscosity higher than around 2, or 1.5 or 1.4 cP at 20°C at room temperature. The aim is more particularly to provide a solution comprising less organic solvent as known solution, or free of, or substantially free of organic solvents. The aim is to provide a liquid solution having less that 30%, or less than 25% or 20% of organic solvent, for inhalation purpose.

These objectives are realized by the means of the invention described in the independent claims, and further detailed in the dependent claims.

The claimed solution solves the above-mentioned drawbacks. In particular, it allows to spread microdroplets of aqueous solutions with a controlled average diameter. It furthermore improves the efficiency of the produced aerosol, which are free or substantially free of undesirable additive or solvent. It further allows to volatilize substances with a minimum energy consumption. The claimed device has thus a longer autonomy and presents an improved comfort of use. The claimed solution allows also to control user taste parameters via the flavour enhancer assay and heat via the temperature of the post heater.

### Brief description of the figures

Example of realization of the presently described invention are illustrated by the following drawings :

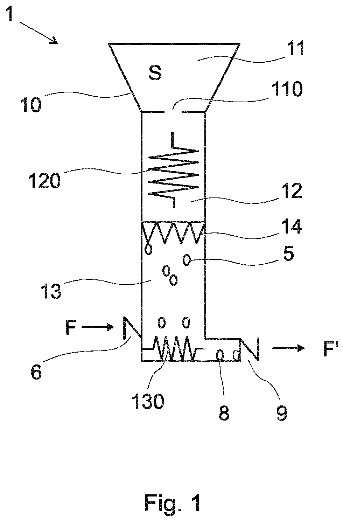

- Fig. 1: schematic view of the device according to the present invention.
- Fig.2: details of the pre-heating chamber of the device according to the present invention.
- Fig.3 : Schematic command diagram for a device according to the present invention.

### Embodiments of the invention

The device **1** of the present disclosure comprises a housing **10** having a storage compartment **11.** The storage compartment **11** may be an internal space of the housing **10,** for example an internal space at an extremity of the housing **10.** To access the storage compartment **11,** the housing **10** comprises a removable part such as a cap or a lid which can be screwed or clipped at the extremity of the housing **10.** Alternatively, a portion of the housing **10** can be separated from the rest of the device to open the access to the storage compartment **11.** Such a removable moiety may represent a portion of the device such as 10% or 20% of its length. It can be for example screwed on the remaining moiety of the housing **10,** representing the body of the device **1.** Alternatively, the storage compartment **11** may be a central compartment of the device which can be accessed by means of an openable lateral lid. Such a lateral lid can be linked to the housing **10** through one or several hinges.

The storage compartment **11** may be adapted to receive a liquid composition **S** from an external tank. Suitable sealings are arranged at the connection between the lid and the housing **10** so as to avoid leakage. According to an embodiment, the housing **10** comprises at least a transparent portion, such as a lateral window, at the level of the storage compartment **11** to allow the user visualizing the level of liquid composition **S** remaining in the storage compartment **11.** The housing portion corresponding to the storage compartment **11** may thus be fully transparent or transparent on a portion of its surface, such as on half of its surface. Alternatively, only the lid covering the storage compartment **11** is transparent.

According to another embodiment, the storage compartment **11** may be adapted to receive a cartridge of a liquid composition **S.** Such a cartridge has an internal volume adapted to store a liquid formulation and a connection part adapted to couple the cartridge to the device **1.** The connection part may comprise a sealing means preventing the leakage of the liquid formulation **S** when the cartridge is separated from the device **1.** It can in addition comprise a fixation means such as a thread or one or several lugs adapted to maintain the cartridge connected to the device **1.** The sealing means of the cartridge is preferably adapted to automatically open once the cartridge is connected to the device 1. To this end. A channel extremity may be present on the device **1,** at the connection point of the cartridge, which can proceed to perforation or opening of the sealing means of the cartridge when coupling it to the device **1.** In case the storage compartment **11** is adapted for receiving a cartridge, it may be free of lid or cap, so that the cartridge can be directly connected to the housing **10** without too many manipulations. Alternatively, a cap may be provided at least to protect the connection part of the device **1.** If present, a cap may also serve at exerting the necessary force to the cartridge for perforation or opening of the sealing means upon coupling to the device **1.** It is here understood that any suitable arrangement can be adapted to the present device **1.**

The storage compartment **11** comprises at least one solution inlet **110** allowing the liquid composition **S** flowing from the storage compartment **11** to a pre-heating chamber **12.** The solution inlet **110** may be a through hole or a plurality of through holes allowing the liquid composition **S** flowing freely to the pre-heating chamber **12.** Such an arrangement may however be sensitive to the gravity and limit the number of positions the device **1** can take under using conditions. The solution inlet **110** may comprise a movable part allowing to close and open it on demand. For example, the pre-heating chamber **12** may have a predetermined volume corresponding to a dose of solution **S** to be inhaled. By opening the solution inlet **110,** the pre-heating chamber **12** can be filled with a suitable volume of liquid solution **S.** Closing the solution inlet **110** before initiating the inhalation then guaranties that no excess of composition **S** is inhaled.

Alternatively, the solution inlet **110** can comprise one or several pores of limited size through which the liquid composition **S** can flow by capillarity. The size and the number of the pores may be adapted to the viscosity of the liquid composition **S.** According to a preferred arrangement, the diameter of the pores is calibrated to prevent or substantially prevent the passage of a liquid having a viscosity higher than around 3 cP or 5 or 10 cP. Alternatively or in addition, the size of the pore is calibrated to allow or substantially allow the progression of a liquid composition by capillarity having a viscosity lower than around 2 cP, or lower than around 1,5 cP, or even lower than 1 cP. According to preferred arrangement, the size of the pores may be calibrated so that the progression by capillarity of a liquid composition having a viscosity lower than 1 cP is 2 or 3 or 4 time faster than the progression of a liquid composition having a viscosity of more than 3 or 4 cP. Alternatively, one or more of the pores of the solution inlet **110** may be larger to allow the free passage of the liquid composition S, independently of its viscosity, and comprise a cotton wick adapted to absorb the liquid composition S by capillarity.

The device **1** of the present disclosure comprises a pre-heating chamber **12** downstream the storage compartment **11.** The pre-heating chamber **12** comprises or is in direct contact with at least one heating device **120.** Such a heating device may be an electrical resistance, or any related device adapted to deliver some heat. The heating device **120** may be placed inside the pre-heating chamber **12** so that it is in direct contact with the liquid composition **S.** It thus allows a very efficient and rapid local heating of the liquid composition **1** surrounding the heating device **120.** The heating device **120** may be positioned closer the solution inlet **110** so that the liquid composition **S** is already warmed when flowing to the pre-heating chamber **12.** Alternatively, the heating device **120** may be placed downstream, closer a volatilizing element so that the liquid composition S is warmed locally just before being spread as microdroplets **5** by the volatilizing element.

The heating device 120 may be encapsulated into a protective housing, which is immerged in the liquid composition **S** inside the pre-heating chamber **12.** Such a protective housing may be of thermo-conductive material such as a metal, like stainless steel, aluminum or similar metallic element. The protective housing may in addition comprise extensions allowing to homogenize the temperature through the volume of the pre-heating chamber **12.** Alternatively, the protective housing may be in clause contact with one or several walls of the pre-heating chamber 12. Alternatively, the heating device **120** may be integrated within one or several walls of the pre-heating chamber **12** such as the lateral walls, or the wall separating the storage compartment **11** from the pre-heating chamber **12,** or the wall separating the pre-heating chamber **12** from an aerosol chamber 13.

According to an embodiment, the heating device **120** is integrated within the solution inlet **110** so that the liquid composition **S** enters in the pre-heating chamber at a suitable temperature.

According to another embodiment, the heating device **120** is integrated to the downstream wall of the pre-heating chamber **12** so that the heating energy is provided at or close to the volatilizing point of the liquid composition **S.**

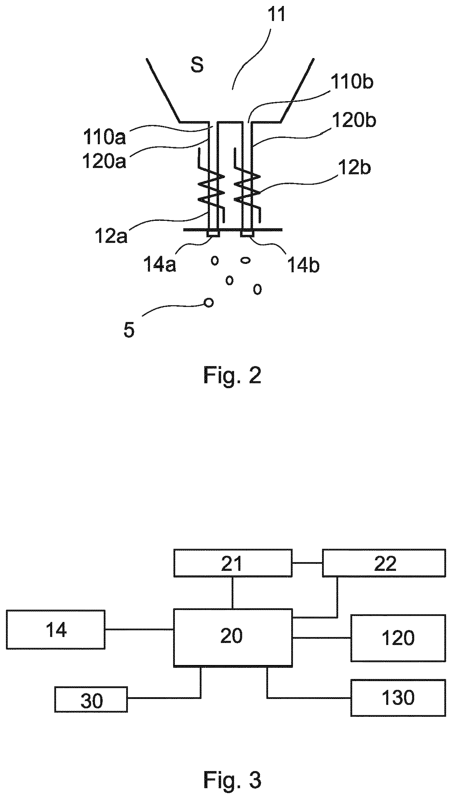

According to an embodiment better shown on figure 2, the pre-heating chamber **12** comprises a plurality of capillaries **12a, 12b** fluidly connected to the solution inlet **110** at one of their extremity and fluidly connected to the volatilizing element at their opposite extremity. The internal diameter of the capillaries **12a, 12b** may be of few hundreds of micrometers. It may be comprised for example between around 100 micrometers and around 800 micrometers, or around 200 micrometers and around 600 micrometers. The internal diameter of the capillaries **12a, 12b** may decrease along their length from the solution inlet **110** to the volatilizing element. This allows to control the progression of the liquid composition S along the capillaries according to its viscosity. The internal diameter of the capillaries **12a, 12b** may then decrease by a value comprised between 10% and 50%, such as around 20% or 30%, from one extremity to the other one. One or several heating devices **120** may surround the capillaries **12a, 12b.** The heating devices **120** can be in direct contact with the capillaries for a fast and efficient temperature control. The heating devices **120** can alternatively be distant from the walls of the capillaries, allowing a smooth temperature variation preventing local burning of the components of the liquid composition **S** or preventing local boiling or pressure increase of the liquid composition **S.** The combination of the heating device **120** and the capillaries having either constant internal diameter or decreasing internal diameters, allows to control the progression speed of the liquid composition **S,** thus influencing its debit.

The heating device **120** is adapted to heat the liquid composition **S** at a temperature which prevents its boiling, the burning of its components, side reactions or chemical degradation thereof. The liquid composition S is thus preferably heated at a temperature comprised between around 30°C and around 90°C, preferably lower than 80°C or 60°C. A temperature comprised between around 40°C and 60°C appears suitable for most of the liquid compositions **S** that are envisaged for the present device **1.** According to another aspect, the heating device **120** is controlled to give the liquid composition **S** the suitable viscosity. The viscosity of the liquid solutions **S,** in particular aqueous solutions, is usually too high at 20°C to provide droplet sufficiently small and/or a suitable debit. Also, the external usage of the device **1,** in particular during wintertime, may occur at very low temperatures, which naturally significantly increase the viscosity of the liquid compositions. The heating device **120** thus help to regulate and reproduce the suitable viscosity with a minimum of energy consumption.

It is highlighted that capillaries **12a, 12b** allow fast temperature exchanges, providing an immediate control of the debit and/or temperature of the liquid solution in the pre-heating chamber **12.** It is also highlighted that the length of the capillaries **12a, 12b** across the pre-heating chamber **12** also influences the temperature control of the liquid composition **S.** The capillaries **12a, 12b** may have a straight shape from the solution inlet **110** to the volatilizing element. For a better temperature control, they can alternatively exhibit a longer pathway in the pre-heating chamber **12** by means of a non-straight shape. The capillaries **12a, 12b** can for example have the shape of a coil or comprise baffles or take any suitable serpentine pathway allowing an efficient temperature exchange with the heating device **12.**

According to an embodiment, the internal volume of the pre-heating chamber **12** comprises a porous material allowing to contain and drain the liquid composition **S.** the heating device **12,** may be arranged as above-mentioned, or inside the porous material comprising the liquid composition **S.** The porous material can thus be maintained at the suitable temperature. Such porous material may be in direct contact with the volatilizing element.

The device **1** according to the present disclosure further comprises a volatilizing element adapted to spread microdroplet **5** toward the aerosol chamber **13** downstream the pre-heating chamber **12** from the liquid composition **S** present in this pre-heating chamber **12.** The volatilizing element comprises at least one mesh **14** through which the liquid composition **S** can pass while forming microdroplets **5.** The pores of the mesh **14** has a diameter preferably comprised between 0.5 micrometers and few micrometers like 3 to 10 micrometers. The mesh **14** may extend through all the surface between the pre-heating chamber **12** and the aerosol chamber **13,** thus presenting a homogenous porosity on the entire surface. Such arrangement appears convenient when the surface between the pre-heating chamber **12** and the aerosol chamber **13** is completely or substantially completely in contact with the liquid composition **S.** It is such a case when the volume of the pre-heating chamber **12** is filled with the liquid composition **S** or when a porous material is in contact with the mesh **14.**

Alternatively, several meshes **14** can be dispatched on the surface separating the pre-heating chamber **2** and the aerosol chamber **13,** forming the only passages allowing the liquid composition **S** passing toward the aerosol chamber **13.** Such a configuration is advantageous in combination with capillaries above described**12a, 12b.** The extremity of the capillaries **12a, 12b** coincides with the corresponding meshes **14.** The liquid composition S, continuously flowing through the capillaries arrives in direct contact with the meshes **14** and can be spread as microdroplets **5** into the aerosol chamber **13.** The number and size of such meshes depends on the needs.

Whether the mesh **14** is a unique mesh covering the surface between the pre-heating chamber **12** and the aerosol chamber **13,** or whether it corresponds to a plurality of small meshes, it may be integrated or combined to a vibrating actuator. For example, the mesh **14** can be integral with a flexible membrane combined to a vibrating actuator. The vibration frequency provided by the vibrating actuator may be comprised between around 50 and 150 kHz. The vibration frequency provided by the vibrating actuator is preferably comprised 80 and 130 KHz, more preferably between 100 and 120 kHz.

The number of holes of the mesh **14,** or of the plurality of meshes is comprised between 500 and 5000, preferably between 800 and 2000. The number of holes spread on the surface between the pre-heating chamber **12** and the aerosol chamber **13** is for example around 1000 holes.

Alternatively, an outlet comprising a vibrating actuator may be arranged upstream the mesh **14** so as to actively direct the liquid composition **S** from the pre-heating chamber toward the aerosol chamber **13** through the mesh **14.** Such a vibrating outlet relates to a pump. The vibration frequency is in this configuration higher than 500 KHz. The frequency may be for example comprised between around 500 and 1000 KHz.

In addition, a mesh **14** may comprise several layers of porous material, either combined together or separated by an interstitial space. The layers of porous material may exhibit different pore size. The pore size of the first layer, placed upstream the liquid pathway can then be larger than the pore size of the last layer, placed downstream. The liquid fragmentation can then be progressive along the pathway, allowing to reduce the applied energy necessary for volatilizing the liquid composition. The vibration frequency can be to this end lowered.

A mesh **14** according to the present disclosure may alternatively or in addition comprise non-flat portions of porous material, so that the contact surface with the liquid composition **S** is increased. The mesh **14** may have a tridimensional shape. It can comprise for example folded sheets.

Pipes, preheating chamber, vibrating mesh chamber and a mesh according to the present disclosure may alternatively or in addition have a smooth surface limiting or avoiding the deposit of residues. It may in addition be coated with a hydrophobic material or an antiadhesive material or any other material limiting the adhesion of residues. Alternatively, the surfaces can be coated with silver, which has in situ bactericide properties.

The vibrating actuator may be any device allowing the vibration of the mesh **14** at the suitable frequencies. It can be for example a piezoelectric or a piezomagnetic actuator or related device. It can in addition comprise several vibrating actuators either identical or different, arranged in parallel.

Downstream the volatilizing element is an aerosol chamber **13** wherein the microdroplets **5** emanating from the volatilizing element are spread. The mean size of the microdroplets **5** is preferably below 4 micrometers, even more preferably below 2 micrometers. It can be around 1 micrometer for a good inhalation. It is indeed an objective of the present device to provide thin microdroplets which can easily flow as far as possible into the lung.

The aerosol chamber **13** can comprise an air inlet **6** allowing to intake external fresh air while inhaling the liquid composition **S.** The air inlet **6** may comprise a valve allowing to open and close it. Such a valve may be tuned to adapt the ratio of fresh air intake compared to the internal aerosol of microdroplets **5.** The ratio of fresh air to microdroplets **5** can be for example around 50/50. Alternatively the inhaled volume comprises more fresh air than microdroplets with a ratio of fresh air to microdroplets 5 higher than 60/40 or even higher 80/20. Alternatively, the inhaled volume comprises less fresh air than microdroplets with a ratio of fresh air to microdroplets 5 lower than 40/60 or even lower than 20/80.

The air inlet **6** may also comprise a filter to avoid inhalation of impurities and microparticles suspended in the air. The aerosol of microdroplet 5 is thus mixt with external air in a predetermine proportion when the user inhales the liquid composition **S.**

The air inlet **6** may be visible on the housing **10** of the device **1** at the level of the aerosol chamber **13.** Alternatively, the air inlet **6** is visible on the housing **10** at the level of the volatilizing element, or at the level of the pre-heating chamber **12,** or at the level of the storage compartment **11.** This means that the external air travels inside the housing **10** of the device **1** from the air inlet **6** to the aerosol chamber **13.** It can for example travel in a double wall around the pre-heating chamber **12.** The intake air can then be pre-heated as well as the liquid composition **S,** due to its proximity with the pre-heating chamber **12.**

The aerosol chamber **13** also comprises a mouth port 9 allowing the user inhaling the liquid composition **S.**

The aerosol chamber **13** may in addition comprise one or several aerosol heater **130.** Any device providing heat, such as an electrical resistance, can be used as such. The aerosol heater **130** may be placed close to the air inlet 6 so as to pre-heat the external air at a suitable temperature, in particular to avoid condensing the microdroplets **5.** Such an arrangement is particularly suitable when the air inlet **6** is directly placed on the aerosol chamber **13,** since it does not flow around the pre-heating chamber **12.** In particular, the aerosol heater can be an electrical resistance, such as a metal or a coil or a metal ceramic heater, with low resistance, preferably comprised between 0.1 and around 2 ohms. the aerosol heater can be more advantageously sub ohm or a metal ceramic heater. Alternatively, the aerosol heater may be of infrared type. Such infrared type heater includes for example the polyimide or carbon infrared heaters, with wave length from around 3 to 14 µm. Infrared type heater having wave lengths corresponding to the water absorbance are preferably used.

Alternatively or in addition an aerosol heater **130** may be placed close to the mouth port 9 to guarantee that the mixture of aerosol droplets and fresh external air is inhaled at the suitable temperature.

Optionally, the aerosol chamber **13** can comprise a means to collect the condensed aerosol which has not been inhaled by the user. The size of the droplets of the aerosols is designed to increase or optimized the inhaled fraction versus the fraction depositing on the surfaces of the device 1. However, a small fraction of aerosol may still condensate on the internal surfaces of the aerosol chamber **13.** The means adapted to collect such a condensed material may take the shape of one or several concave wall arranged at a bottom position so as to collect condensed fluids. The bottom position designates the lowest part of the device **1,** either at rest or during its use or both. The means to collect the condensed fluids may further comprise one or several capillaries connected at the concave wall in a way to flow these condensed fluid back to an upstream part of the device 1. The capillaries of the collecting means can then join the concave wall of the aerosol chamber **13** to the volatilizing element. The collected condensed fluids are thus guide through the vibrating elements and volatilized again. Alternatively or in addition, the capillaries of the collecting means join the pre-heating chamber **12.** The retrieved condensed fluids can thus be heated again at the proper temperature and volatilized again. Alternatively or in addition, the capillaries of the collecting means join the storage compartment **11.** By this way, the retrieved condensed fluid is driven back to the full circuit.

The overall design of the present device can allow to remove one or several of its elements. It can be for example modular, having a first removable part comprising the storage compartment, a second removable part related to the pre-heating chamber **12,** a third removable part corresponding to the volatilizing element, and a fourth part related to the aerosol chamber **13.** By this way, any of these modular parts can be independently separated from the others and clean up and/or replaced. According to a specific embodiment, the volatilizing element, or at least the meshes **14** it comprises, can be easily dissociated from the device **1** by the user so as to be cleaned up or replaced by a new one. This facilitates the maintenance of the device **1** by the user and allows him to maintain the proper volatilization conditions of the solution **S,** without degradation of the taste due to potential accumulation of residues. It is however highlighted that due to the absence of burn, the device **1** normally does not contain residues of liquid degradation or burn.

Alternatively or in addition, the device **1** can allow one or several modes related to either the disinfection or the cleanup of at least some of the parts of the device **1** using a dedicated cleaning solution. Such a cleaning solution could be for example an acidic solution, or a basic solution, an antiseptic solution, an alcoholic solution or a selection of such solutions to be used in a precise order. In a cleaning mode, the cleaning solution can be included to the storage compartment 11 for a circulation in the complete circuit of the device.

The device according to the present disclosure further comprises a command input **22** such as an input button or a combination of input button, or a tactile display, or any related input command that a user can activate. The command input is connected to a command unit **20** adapted to modulate the heating device **120** of the pre-heating chamber **12.** The command unit **20** may in addition allow the modulation of the frequencies of the vibrations of the mesh **14** of the volatilizing element. The command unit **20** may in addition allow the modulation of the aerosol heater **130** or the plurality of aerosol heaters. The command unit may in addition allow to modulate the fresh air intake by closing more or less the air inlet **6.** The device **1** can in addition comprise a display **21** allowing the user to input data and/or receive information on the status of the device **1.** The device **1** may in addition comprise a communication unit **30** allowing to share some data with a remote system. Such a remote system may be for example a platform providing services or information to the user of the device, such as notice of use, or specific settings with regard to the liquid compositions **S.** The remote system may allow to automatically update the command unit **20.** Such updates can for example relate to better processes involved in the mutual management of the heating device **120** and the frequency of vibrations of the volatilizing element. The remote system may alternatively designate a mobile phone or another terminal of the user, such as his computer.

The input command **22** can allow the user to choose a debit or a predetermined quantity of aerosol **5** or any other parameters. For ease of use, the command unit **20** may automatically select the proper combination of temperatures of the heating device **120,** aerosol heater **130** and frequency of vibration of the volatilizing element to provide the user the requested results. In addition, the command unit **20** may automatically select the proper combination of temperatures of the heating device **120** and frequency of vibration of the volatilizing element so that a minimum energy is consumed. For example, heating of the heating device **120** lower than necessary to provide the suitable viscosity of the liquid composition **S** and compensated by an increase of the vibration frequency of the volatilizing element if the global energy consumption appears lower for a given result. The activation of the heating device **120** and the volatilizing element may also be adapted according to the autonomy of the device **1.** It is here highlighted that the device 1 can comprise a battery and a plugging means to recharge the battery.

The input command 22 may in addition allow to select a cleanup mode or a maintenance mode or any other specific mode of the device **1.**

The device according to the present disclosure thus allows to provide aerosol having thin microdroplets based on liquid compositions S having a relatively high viscosity. It furthermore allows such a microdroplet generation with a minimum of energy consumption thanks to the combination of the heating element **120** and the vibrating mesh **14.** Liquid compositions having high viscosity necessitate stronger vibrations to produce microdroplets, thus consuming more energy. Even under such conditions, the size of the produced microdroplets may not be so homogenous or so thin compared to the present aerosol, which comprises a pre-heating chamber 12 upstream the volatilization element.

The present invention further relates to a kit comprising a device **1** and at least one liquid composition **S.** The liquid composition **S** may be separately packed in a tank adapted to fill or refill the storage compartment **11.** The liquid composition **S** may be separately packed in a cartridge adapted to be connected to the device **1.** Such a cartridge may comprise a readable device such as a RFID or any similar device allowing to automatically identify the nature of the liquid composition, including its viscosity at a given temperature, and/or its expiration date. Other characteristics may be stored in the readable device. The readable device may be automatically read by the device **1** so that the proper settings are suggested to the user through the display **21** or automatically applied. The kit can comprise additional liquid compositions **S,** offering a large variety of choice to the user. In case the liquid composition comprises a medical treatment or any pharmaceutical compounds, the kit can comprise in addition the corresponding notice of use.

The liquid compositions **S** of the present invention are preferably aqueous compositions. The liquid composition **S** thus comprise more that around 60% by weight of water, preferably more that 70% by weight or even more preferably more than 80% by weight of water, this amount being determined with regard to the total weight of the liquid compositions S. The water may be mineral water, distilled water, pure water, or water having pharmaceutical quality such as water for injection. To this end, the complete liquid compositions S may be subjected to sterilization processes.

The liquid compositions **S** of the present invention further comprises one or more substances having biological effect through inhalation. Such substances include for example Ventolin or any other drugs commonly used to treat respiratory symptoms or diseases like asthma, as well as acceptable salts thereof. The substance of the liquid compositions S may also be or include nicotine, also known as Pyridine-3-(1-methyl-2-pyrrolidinyl). The substance may designate nicotine derivatives or salts thereof. Nicotine derivatives include for example 4-nitronicotine-N-oxide, 4-aminonicotine, nornicotine, anabasine, myosmine, nicotirine, and anatabine. The nicotine or nicotine derivatives may be combined to a salt selected from the group of sulfate, tartrate, Hexafluorosilicate, hydrochloride, diformate, triacetate, tripropionate, tributyrate, isovalerate, dinitrophenolate, dipicrate, dihydrochloride, hydroiodide, picrolonate, phthalate, dipicrolonate, aurichloride, benzoic, levunilic, pyruvic or a combination thereof.

The substance may alternatively be or comprise a natural oil or a natural plant extract. For example, the substance may be a cannabinoid such as Tetrahydrocannabinol (THC), Cannabidiol (CBD), Cannabinol (CBN), Cannabigerol (CBG), Tetrahydrocannabivarin (THCV), cannabidivarin (CBDV), Cannabichromene (CBC).

The substance of the liquid compositions S may be comprised between 1% by weight and 10% by weight of composition.

The liquid compositions **S** may further comprise a polyol, in particular a diol. Such polyol may be selected in the group of propylene glycol (propane-1,2-diol), vegetol (Propane-1 3 diol), glycerine (propane-1,2,3-triol), Propane 1-1-diol, Propane 2-2 diol, Butane 1,4 diol, Butane 1,3 diol, Butane 2,3 diol and Pentane 1,5 diol or a mixture thereof. The amount of the polyol is comprised between 3 and 40%, preferably between 5 and 35% by weight of liquid composition **S.**

To transfer the flavor from water solution, a flavor enhancer may be required. Such polyol may be used as flavor enhancer. Preferred polyols used as flavour enhancer can be selected among the propylene glycol (PG), the vegetal glycerin (VG), and the glycerin. For example, a combination of propylene glycol (PG) and vegetal glycerin (VG) can be used in a ratio of PG/VG of around 80/20, around 70/30, around 60/40, around 50/50 can be used. Some solution having more VG than PG can also be used, such a ratio PG/VG of around 40/60, around 30/70 or around 40/60.

For e-cigarette, aqueous solutions may not transmit taste of nicotine and aroma in a proper manner. To improve the transmission of the taste to the user, a combination of physical particle size reduction and a flavor enhancer, which is also used as surfactant, can be used. The combination of particle size reduction and flavor enhancer may thus be required to properly transmit the taste.

The liquid composition **S** of the present invention may comprise one or several flavors. It may in addition comprise preservative compounds such as bactericides (triacetine), fungicides, and anti-oxidants.

The present invention further comprise a method of vaporizing an liquid solution into an aerosol adapted for inhalation, the liquid composition S having a viscosity higher than 2 or 3 or 5 Cp at 20°C. The present method comprises a heating step a) of continuously and instantaneously pre-heating a liquid solution S at a temperature lower that its boiling point, so that no vapor is produced and no product degradation occurs. The pre-heated solution S is directly submitted to a spreading step b) of volatilizing the pre-heated liquid solution **S** into microdroplets **5** having an average diameter of less than 4 µm, like around 1 µm.

The steps a) and b) preferably occurs concomitantly. They may be manually initiated by the user by means of a command button **22.**

The method of the present invention further comprises a modulation step c) of modulating one or more of the temperature of the pre-heating chamber **12,** the frequency of vibration of the volatilizing element, and the temperature of the aerosol heater. The modulation step c) includes a debit selection by the user and the automatic modulation of the heating device **120** and the frequency of vibration of the volatilizing element by the command unit **20.** In case several vibrating actuators **14** are present, the modulation step c) may allow to sequentially activate the vibrating actuators to increase or reduce the flowrate of the liquid composition toward the aerosol. One or several vibrating actuators can be independently activated.

### Reference numbers and symbols

- 1: Device
- 10: Housing
- 11: Storage compartment
- 110: Solution inlet
- 12: Preheating chamber
- 120: Heating device
- 13: Aerosol chamber
- 130: Aerosol heater
- 14: Vibrating mesh
- 20: Command Unit
- 21: Displa
- 22: Command Button
- 30: Communication unit
- 5: Droplets
- 6: Air inlet
- 8: Heated aerosol
- 9: Mouth port

## Claims

1. Device (1) comprising a housing (10) having a storage compartment (11) adapted for the storage of a liquid compositions (S), a volatilizing element comprising a vibrating mesh (14) adapted to volatilize the aqueous solution (S) into microdroplets (5), and an aerosol chamber (13),
**characterized in that** the device further comprises a pre-heating chamber (12) downstream said storage compartment (11) and in fluid communication with said storage compartment (11) by means of at least one solution inlet (110), said at least one pre-heating chamber (12) comprising one or more heating device (120) and being arranged upstream said volatilizing element.

2. Device according to claim 1, said heating device being adapted to heat the liquid composition (S) at a temperature lower than its boiling point so that it provides no increase, or substantially no increase of pressure.

3. Device according to one of the preceding claims, said vibrating mesh (14) comprises one or several flat porous membranes having pore size of around 1 µm or lower, combined with at least one piezoelectric or magnetoelectric actuator.

4. Device according to one of claims 1 to 3, wherein said volatilizing element is adapted to vibrate at frequencies comprised between around 50KHz and around 150KHz or between around 500KHz and around 1000 KHz.

5. Device according to one of claims 1 to 4, wherein said volatilizing element comprises a non-flat porous material adapted to present an increase contact surface with said liquid composition (S) and/or a multilayer porous material.

6. Device according to one of claims 1 to 5, wherein said pre-heating chamber (12) comprises one or several capillaries or a porous material driving the liquid composition (S) in contact to said volatilizing element.

7. Device according to one of claims 1 to 6, further comprising an aerosol chamber (13), downstream the volatilizing element, said aerosol chamber comprising a mouth port (9) and an air inlet (6) adapted to combine the microdroplets (5) with external air under inhalation via the mouth port.

8. Device according to claim 7, wherein the aerosol chamber (13) further comprises at least one aerosol heater (130).

9. Device according to one of claims 1 to 8, further comprising a command input (22) adapted for modulating the temperature of the pre-heating chamber (12), the frequency of vibration of the volatilizing element or both of the temperature of the pre-heating chamber (12) and the frequency of vibration of the volatilizing element.

10. Method of volatilizing a liquid composition (S) into microdroplets (5) having a diameter lower than 4 micrometers, the liquid composition having a viscosity higher than 2 cP at 20°C, the method comprising a heating step a) of pre-heating said liquid composition (S) at a temperature lower that its boiling point, so that no vapor is produced, and a spreading step b) of volatilizing the pre-heated liquid composition into microdroplets (5) having an average diameter lower than around 4 µm.

11. Method according to claim 10, further comprising the modulation step c) of modulating the temperature of the pre-heating chamber (12) and/or the frequency of vibration of the vibrating mesh and/or the number of vibrating actuators when applicable.

12. A kit comprising one or more liquid compositions (S) separately packed in a recipient or a cartridge and a device as described in the claims 1 to 11.

13. A kit according to claim 12 further comprising a written notice of use of the liquid composition (S) and/or the device (1).

14. A liquid composition comprising more than 60% in weight of water and having viscosity higher than 2cP at 20°C, between 1 and 10% of a substance having a biological effect under inhaling conditions, and between 5 to 40% of a polyol.

15. The liquid composition (S) according to claim 14, being substantially free of organic solvent.

16. The liquid composition according to claim 1 to 15, further comprising one or several flavours.

17. Liquid composition according to one of claims 14 to 16, wherein said substance is selected from nicotine, a nicotine derivative, Tetrahydrocannabinol (THC) or cannabinoid family or a pharmaceutically acceptable salt thereof.
